# EUROPEAN PATENT APPLICATION

(11) **EP 4 744 717 A1**
(43) Date of publication of application: **20.05.2026**
(21) Application number: 25209322.4
(22) Date of filing: 17.10.2025
(51) Int. Cl.: A61M 39/14, B29C 65/78, B29C 65/00, B29C 65/20, A61M 39/18

(54) **SERIAL BLADE LOADING FOR STERILE CONNECTION DEVICE**

(30) Priority: 19.11.2024 US 202463722105 P
(71) Applicant: Fenwal, Inc., Lake Zurich, IL 60047 (US)
(72) Inventor: MADSEN, James G., Lake Zurich, 60047 (US)
(74) Representative: Maikowski & Ninnemann Patentanwälte Partnerschaft mbB

(57) **Abstract**

A sterile connection device includes first and second carriages, each defining a portion of a first dock and a portion of a second dock. A first blade is associated with the first dock, while a second blade is associated with the second dock. When a pair of tubes is received in only the first dock, the tubes are cut using the first blade, then joined, and then a single replacement blade is moved into association with the second dock, which moves the second blade into association with the first dock and removes the first blade from association with the first dock. When a pair of tubes is received in each dock, the tubes are cut using the first and second blades, then joined, and then two replacement blades are moved into association with the docks, which moves the first and second blades out of association with the docks.

## Description

### Background

### Field of the Disclosure

The present disclosure relates to joinder of tubing. More particularly, the present disclosure relates to systems and methods for sterilely joining tubing.

### Description of Related Art

Fluid flow systems or assemblies that are pre-sterilized and/or preassembled are used in a wide variety of medical and non-medical applications. Medical applications may include, for example, administration of medical fluids to a patient for therapeutic and/or diagnostic purposes, blood and/or blood component or other cell collection or processing, dialysis, and other medical procedures. Non-medical applications for such systems or assemblies may include, for example, pharmaceutical manufacturing and cell processing. In the medical field in particular, such flow systems commonly employ one or more pre-filled containers or other sources of medical fluid or agent and an associated fluid flow circuit or system (sometimes called a tubing set) containing the necessary flow tubing, valves, flow controllers, process chambers, and the like to carry out the particular procedure, either alone or in cooperation with a reusable controller or other device. It is not unusual, for example, for a medical fluid flow system to include or be used in association with a container of a suitable drug, saline, anticoagulant, dextrose solution, sterile water, cell preservative, or the like, to name just a few examples.

Such a fluid flow system can, however, pose manufacturing or assembly challenges for different reasons. One reason can be that the pre-filled containers of medical liquid, powder, or other agent that is administered to the patient or otherwise employed in the medical fluid flow system, require different sterilization techniques than other portions of the fluid flow system. For example, empty plastic tubing, containers, flow control devices, and/or processing devices or chambers, which do not contain any substantial amount of liquid or other agent, may be sterilized with gamma or electron beam (e-beam) radiation or by exposure to a sterilizing gas, e.g., ethylene oxide. However, gas sterilization would be ineffective to sterilize an agent, such as a liquid, powder, or drug, contained in a sealed container, and exposing the agent to ionizing radiation may degrade or otherwise have a deleterious effect on the agent. Also, there may be situations where different portions of a sterile fluid flow system, even though suitable for the same sterilization process, are separately manufactured and sterilized for other reasons and then subsequently assembled in a sterile manner.

In addition, sterile connections often need to be made on-site, by the end user, e.g., at the location where the fluid flow systems are being used to treat patients or collect or process blood or blood components or biologic materials, or in other therapeutic or diagnostic procedures. As a result, a number of different approaches have been used in assembling sterile fluid flow systems. For example, one technique for manufacturing such systems employs the use of a sterile docking system, such as a device disclosed in U.S. Patent No. 4,157,723, which is hereby incorporated herein by reference. As illustrated therein, the sterile docking system comprises a pair of mating members, each having a facing membrane. One of the mating members is connected to a pre-sterilized container of liquid, drug or other agent and the other mating member is attached to a pre-sterilized fluid flow system, which may include one or more empty containers. After the two members are joined, the docking system is exposed to radiant energy, causing the membranes to melt and form a sterile fluid pathway through the mating members. Fluid may then be transferred from the initial container into an empty container in the fluid flow system, and the flow path sealed and severed. The initial container and mating members are then discarded. While this works satisfactorily, it entails multiple manufacturing steps of transferring solution from one container to another in a sterile manner and the associated quality control procedures with such a step. It also requires the disposal of a portion of the product with increased product and waste cost.

According to an alternative approach, which is described in U.S. Patent No. 4,978,446 (which is hereby incorporated herein by reference), sterilizing filters are used on the inlet flow line that couples a pre-sterilized liquid container or the like to a separately sterilized fluid flow tubing system. In this approach, medical personnel are required to manually join the fluid flow tubing system to the fluid container, such as by spiking the fluid container with a piercing member associated with the fluid flow system. In addition to the administrative requirements for individually ordering, storing, and prescribing solutions and disposable flow systems or sets, there is the added possibility of errors, such as by connection of a container of an incorrect liquid or other agent or an improper flow system to be used in association with the procedure.

There are also known devices commonly referred to as sterile tubing welders or sterile connection devices, with the devices described in U.S. Patent No. 11,731,371 (which is hereby incorporated herein by reference) being exemplary. The devices described in U.S. Patent No. 11,731,371 use a heated blade to cut the ends off of a pair of tubes to present an exposed end of each tube. The exposed ends of the tubes are subsequently joined together after the blade is removed so as to provide a joined tube. After the tubes have been joined, the blade and the ends of the tubes that have been cut off are disposed of as waste products of the procedure.

Notably, U.S. Patent No. 11,731,371 describes an embodiment of a sterile connection device in which the ends of two pairs of tubes are simultaneously cut off, with each pair of tubes thereafter being joined together to provide two pairs of joined tubes. While the device is configured for simultaneously joining two pairs of tubes, it is also capable of joining a single pair of tubes. One option for enabling this functionality is to provide a relatively long ("two-weld") blade that is capable of being used to simultaneously cut the ends off of two pairs of tubes. While such a "two-weld" blade is suitable for cutting the ends off of a single pair of tubes, it is larger than necessary, thus producing excess waste and incurring excess material cost compared to the waste created and the cost incurred when using a shorter ("one-weld") blade that is specially configured to cut the ends off of only a single pair of tubes. An alternative approach would be to load the sterile connection device with both "one-weld" blades and "two-weld" blades, with the appropriate blade being selected and dispensed, depending on the number of tubes to be joined. However, the controls and components required to allow for differently sized blades to be selectively deployed may be complicated and expensive. Accordingly, it would be advantageous to provide an approach that enables a single sterile connection device to be used to selectively join either one pair of tubes or two pairs of tubes without resulting in the above-noted disadvantages.

### Summary

There are several aspects of the present subject matter which may be embodied separately or together in the devices and systems described and claimed below. These aspects may be employed alone or in combination with other aspects of the subject matter described herein, and the description of these aspects together is not intended to preclude the use of these aspects separately or the claiming of such aspects separately or in different combinations as set forth in the claims appended hereto.

In one aspect, a sterile connection device includes a housing, a first carriage, and a second carriage. The first carriage includes a first lower jaw defining a first portion of a first dock and a first portion of a second dock, and a first upper jaw configured to move between an open condition spaced away from the first lower jaw and a closed condition positioned adjacent to the first lower jaw. The second carriage is positioned laterally of the first carriage and includes a second lower jaw defining a second portion of the first dock and a second portion of the second dock, and a second upper jaw configured to move between an open condition spaced away from the second lower jaw and a closed condition positioned adjacent to the second lower jaw. The sterile connection device also includes a blade handling assembly configured to move cutting blades into a plurality of positions within the housing, a blade heating assembly, and a system controller. The system controller is programmed to execute a single-pair sterile connection procedure when a single pair of tubes is received by the first dock, no tubes are received by the second dock, a first cutting blade is associated with the first dock, a second cutting blade is associated with the second dock, and the first and second upper jaws are in the closed conditions. The system controller is also programmed to execute a double-pair sterile connection procedure when a first pair of tubes is received by the first dock, a second pair of tubes is received by the second dock, the first cutting blade is associated with the first dock, the second cutting blade is associated with the second dock, and the first and second upper jaws are in the closed conditions. The single-pair sterile connection procedure includes controlling the blade handling assembly, the blade heating assembly, and at least one of the first and second carriages to cut the single pair of tubes using the first cutting blade and then join the single pair of tubes. The system controller then controls the blade handling assembly to advance a single replacement cutting blade in a longitudinal direction into association with the second dock, thereby advancing the second cutting blade in the longitudinal direction out of association with the second dock and into association with the first dock and advancing the first cutting blade in the longitudinal direction out of association with the first dock. The double-pair sterile connection procedure includes controlling the blade handling assembly, the blade heating assembly, and at least one of the first and second carriages to cut the first pair of tubes using the first cutting blade and then join the first pair of tubes and to cut the second pair of tubes using the second cutting blade and then join the second pair of tubes. The system controller then controls the blade handling assembly to advance a first replacement cutting blades in a longitudinal direction so as to place the first replacement cutting blade into association with the second dock, thereby advancing the second cutting blade in the longitudinal direction out of association with the second dock and into association with the first dock and advancing the first cutting blade in the longitudinal direction out of association with the first dock. Next, the system controller controls the blade handling assembly to advance a second replacement cutting blade in the longitudinal direction so as to place the second replacement cutting blade into association with the second dock, thereby advancing the first replacement cutting blade in the longitudinal direction out of association with the second dock and into association with the first dock and advancing the second cutting blade in the longitudinal direction out of association with the first dock.

In another aspect, a sterile connection device includes a housing, a first carriage, and a second carriage. The first carriage includes a first lower jaw defining a first portion of a first dock and a first portion of a second dock, and a first upper jaw configured to move between an open condition spaced away from the first lower jaw and a closed condition positioned adjacent to the first lower jaw. The second carriage is positioned laterally of the first carriage and includes a second lower jaw defining a second portion of the first dock and a second portion of the second dock, and a second upper jaw configured to move between an open condition spaced away from the second lower jaw and a closed condition positioned adjacent to the second lower jaw. The sterile connection device also includes a blade handling assembly configured to move cutting blades into a plurality of positions within the housing, a blade heating assembly, and a system controller. The system controller is programmed to execute a single-pair sterile connection procedure when a single pair of tubes is received by the first dock, no tubes are received by the second dock, a first cutting blade is associated with the first dock, a second cutting blade is associated with the second dock, and the first and second upper jaws are in the closed conditions. The system controller is also programmed to execute a double-pair sterile connection procedure when a first pair of tubes is received by the first dock, a second pair of tubes is received by the second dock, the first cutting blade is associated with the first dock, the second cutting blade is associated with the second dock, and the first and second upper jaws are in the closed conditions. The single-pair sterile connection procedure includes controlling the blade handling assembly, the blade heating assembly, and at least one of the first and second carriages to cut the single pair of tubes using the first cutting blade and then join the single pair of tubes, while the double-pair sterile connection procedure includes controlling the blade handling assembly, the blade heating assembly, and at least one of the first and second carriages to cut the first pair of tubes using the first cutting blade and then join the first pair of tubes and to cut the second pair of tubes using the second cutting blade and then join the second pair of tubes. The system controller is further programmed to receive a single blade replacement input in connection with executing the single-pair sterile connection procedure, with the single blade replacement input instructing the system controller to control the blade handling assembly to advance a single replacement cutting blade in a longitudinal direction into association with the second dock, thereby advancing the second cutting blade in the longitudinal direction out of association with the second dock and into association with the first dock and advancing the first cutting blade in the longitudinal direction out of association with the first dock. The system controller is also programmed to receive a double blade replacement input in connection with executing the double-pair sterile connection procedure, with the double blade replacement input instructing the system controller to control the blade handling assembly to advance a first replacement cutting blade in a longitudinal direction so as to place the first replacement cutting blade into association with the second dock, thereby advancing the second cutting blade in the longitudinal direction out of association with the second dock and into association with the first dock and advancing the first cutting blade in the longitudinal direction out of association with the first dock, and control the blade handling assembly to advance a second replacement cutting blade in the longitudinal direction so as to place the second replacement cutting blade into association with the second dock, thereby advancing the first replacement cutting blade in the longitudinal direction out of association with the second dock and into association with the first dock and advancing the second cutting blade in the longitudinal direction out of association with the first dock.

### Brief Description of the Drawings

Fig. 1 is a perspective view of a sterile connection device according to an aspect of the present disclosure, upper jaws of the device in a closed condition;
Fig. 2 is a perspective view of the sterile connect device of Fig. 1, with first and second carriages of the device aligned and upper jaws of the carriages in an open condition;
Figs. 3-5 are perspective views of the sterile connect device of Fig. 1, with various portions thereof broken away or omitted for illustrative purposes;
Fig. 6 is a top plan view of a disposable blade used in combination with the sterile connect device of Fig. 1;
Fig. 7 is a side elevational view of a blade cartridge configured for dispensing blades of the type shown in Fig. 6;
Fig. 8 is a perspective view of the blade cartridge of Fig. 7;
Fig. 9 is a perspective view of the sterile connection device of Fig. 1, with the upper jaws of the first and second carriages in a closed condition;
Fig. 10 is a perspective view of the sterile connection device of Fig. 1, with the first and second carriages out of alignment and the upper jaws in their closed condition;
Fig. 11 is a perspective view of the sterile connection device of Fig. 1, with the first and second carriages out of alignment and the upper jaws in their open condition;
Figs. 12A-12C are diagrammatic views illustrating a blade replacement protocol following joinder of a single pair of tubes; and
Figs. 13A-13D are diagrammatic views illustrating a blade replacement protocol following joinder of two pairs of tubes.

### Description of the Illustrated Embodiments

The embodiments disclosed herein are for the purpose of providing a description of the present subject matter, and it is understood that the subject matter may be embodied in various other forms and combinations not shown in detail. Therefore, specific designs and features disclosed herein are not to be interpreted as limiting the subject matter as defined in the accompanying claims.

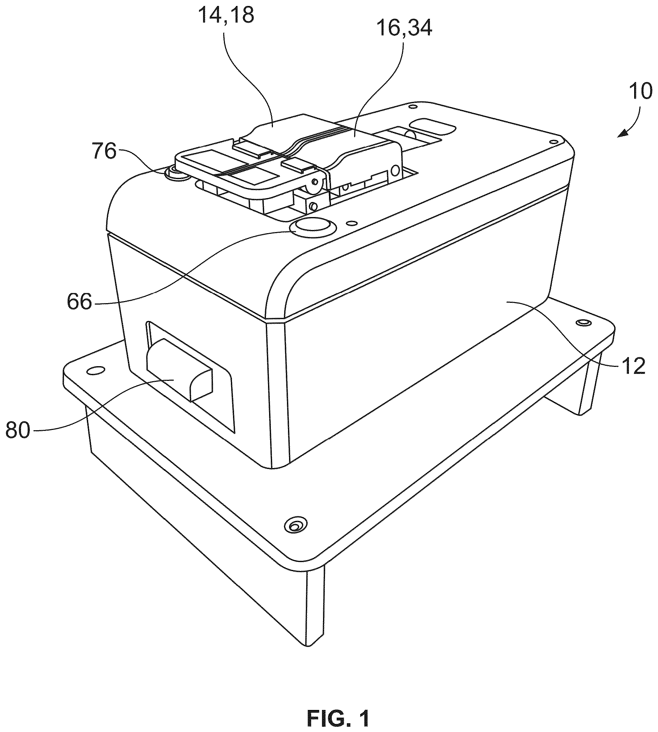

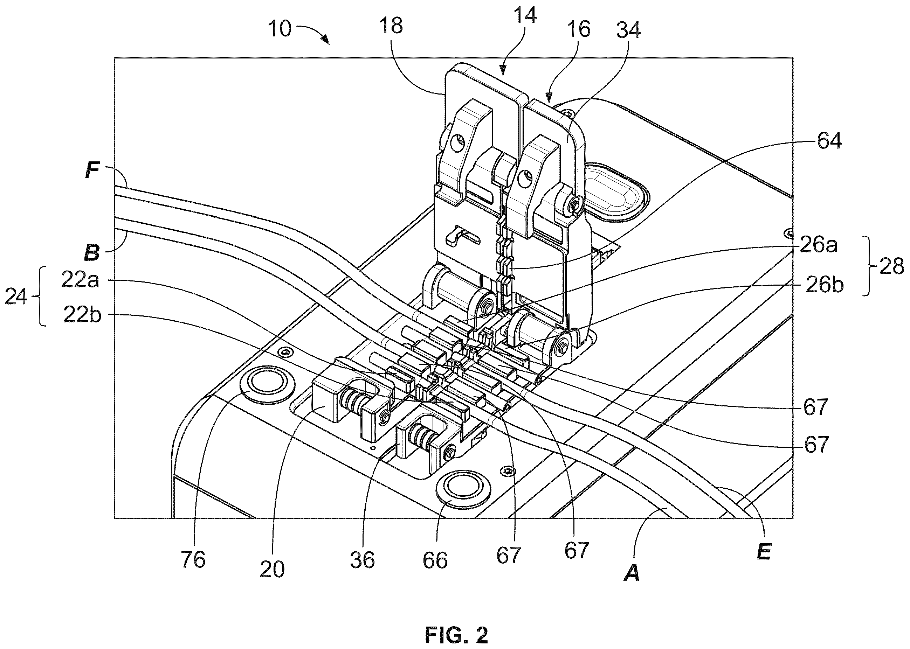

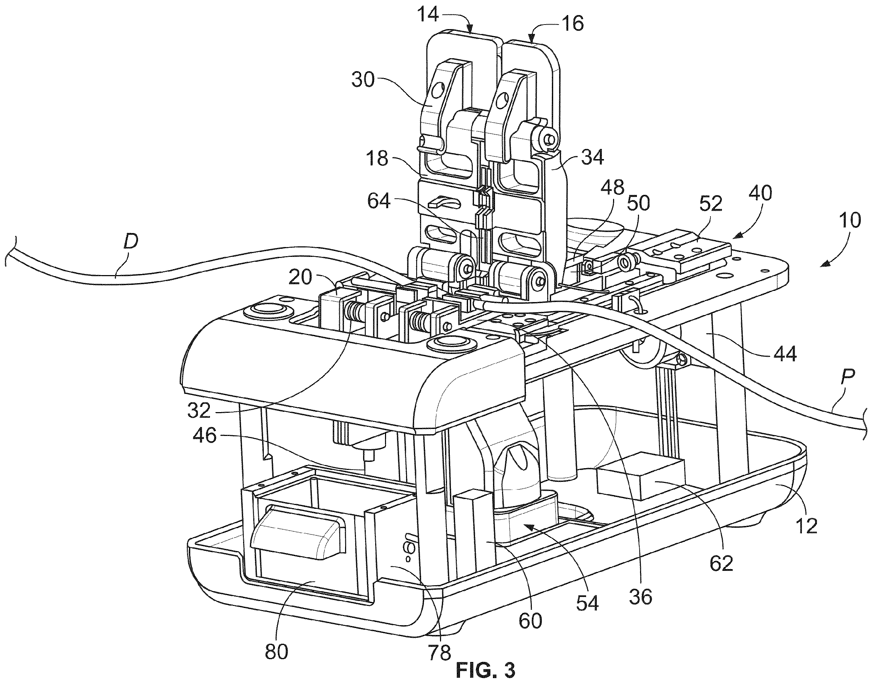

Figs. 1 and 2 illustrate an exemplary embodiment of a sterile connection device 10 according to an aspect of the present disclosure, while Figs. 3-5 illustrate the sterile connection device 10 with assorted portions thereof broken away or omitted for illustrative purposes. While sterile connection devices according to the present disclosure are particularly well-suited for sterile connection of tubes formed of polyvinyl chloride, it is within the scope of the present disclosure for the sterile connection device 10 to be used to sterilely connect tubes formed of other materials.

The illustrated sterile connection device 10 includes a housing 12 containing the various components of the sterile connection device 10. The housing 12 may be variously configured without departing from the scope of the present disclosure, which may include the housing 12 having different portions being formed of, for example, a metallic material, a plastic material, or a combination of metallic and plastic materials.

In the illustrated embodiment, first and second carriages 14 and 16 are associated with an upper surface or face of the housing 12, with the second carriage 16 being positioned laterally of the first carriage 14. At least a portion of at least one of (but more preferably both of) the carriages 14, 16 is movable with respect to the housing 12 during a sterile connection procedure in which either one pair of tubes "P" and "D" (Fig. 3) or a first pair of tubes "A" and "B" and a second pair of tubes "E" and "F" (Fig. 2) are joined together. The first carriage 14 includes an upper jaw 18 and a lower jaw 20, with the upper jaw 18 being movable between a closed condition (Fig. 1) and an open condition (Figs. 2 and 3). The upper jaw 18 of the illustrated embodiment is pivotal between the closed and open conditions, but it should be understood that the upper jaw 18 may be otherwise movable between the closed and open conditions (e.g., via vertical translational movement) without departing from the scope of the present disclosure.

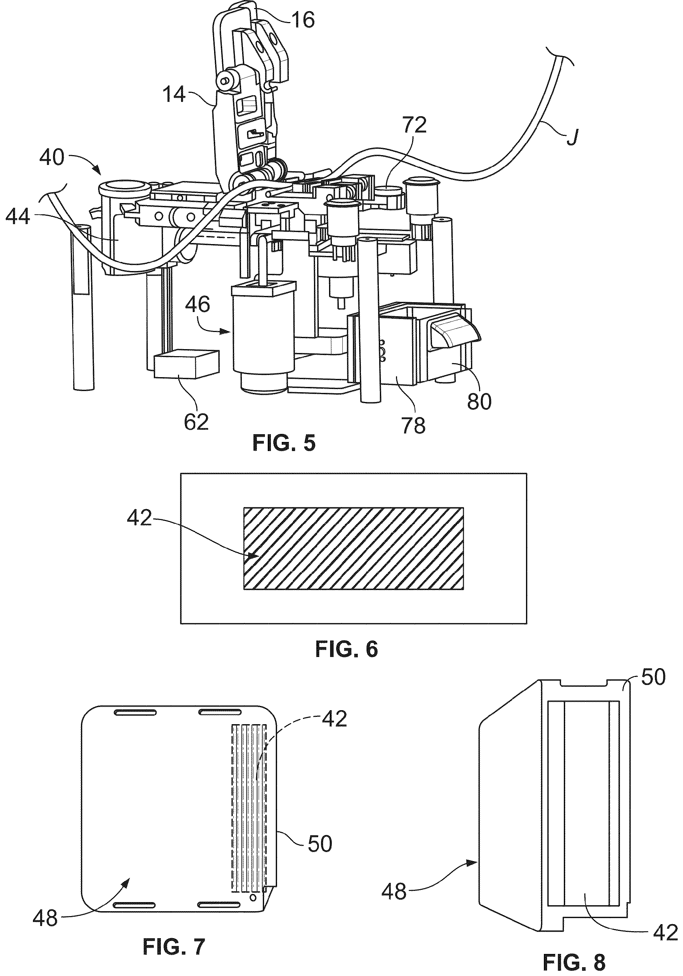

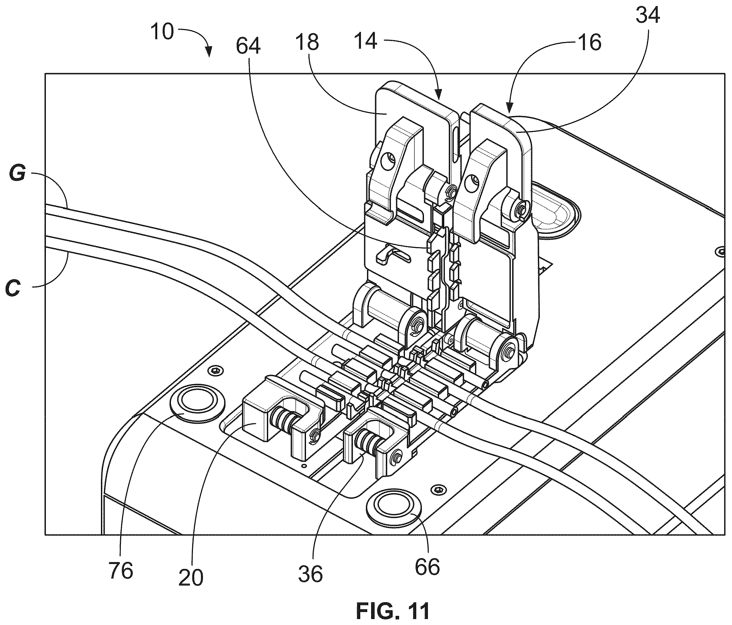

When the upper jaw 18 is in the open condition, the lower jaw 20 is exposed or uncovered, which allows either a single pair of sealed tubes P and D (Fig. 3) or a first pair of tubes A and B and a second pair of tubes E and F (Fig. 2) to be mounted within the first carriage 14 at the beginning of a sterile connection procedure. The term "single-pair" is used herein to refer to a sterile connection procedure in which the sterile connection device 10 is used to join a single pair of tubes (as in Figs. 3-5), while the term "double-pair" is used herein to refer to a sterile connection procedure in which the sterile connection device 10 is used to join a first pair of tubes and a second pair of tubes (as in Figs. 2 and 9-11). The upper jaw 18 is in its open condition at the end of a sterile connection procedure, allowing for a single joined tube "J" (Fig. 5) to be removed from the first carriage 14 at the end of a "single-pair" sterile connection procedure and for a first joined tube "C" and a second joined tube "G" (Fig. 11) to be removed from the first carriage 14 at the end of a "double-pair" sterile connection procedure.

The lower jaw 20 of the first carriage 14 defines a first portion 22a of a first dock 24 and a first portion 26a of a second dock 28 (Fig. 2). In the illustrated embodiment, the first portions 22a and 26a of the first and second docks 24 and 28 are substantially identical, but it should be understood that they may be differently configured without departing from the scope of the present disclosure. The first portion 22a, 26a of each dock 24, 28 defines a first portion of a proximal slot and a first portion of a distal slot that is parallel to the first portion of the proximal slot. The first portion of each slot is sized and configured to accommodate a portion of a tube that is to be joined to a tube received by the other slot of the same dock (i.e., a tube received within the first portion of the proximal slot of the first dock 24 is configured to be joined to a tube received within the first portion of the distal slot of the first dock 24, while a tube received within the first portion of the proximal slot of the second dock 28 is configured to be joined to a tube received within the first portion of the distal slot of the second dock 28). This may include the first portion of each slot having a general arcuate or V-shaped profile to facilitate proper positioning (namely, centering) of a tube inserted into the slot.

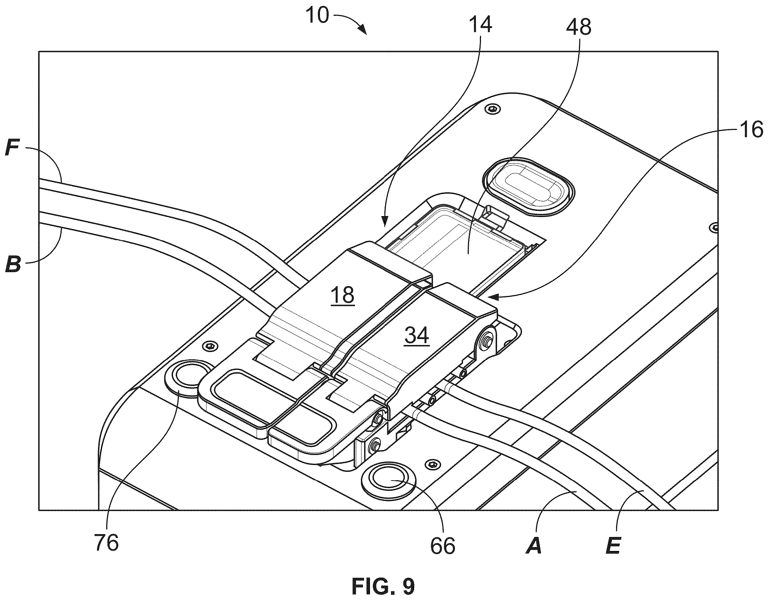

When the upper jaw 18 of the first carriage 14 is in its closed condition (Fig. 9), it covers or overlays tubes received within the slots to retain the tubes in position during a sterile connection procedure. The illustrated upper jaw 18 includes a latch 30 (Fig. 3) that is configured to engage a pin 32 of the lower jaw 20 when the upper jaw 18 is in the closed condition. Such an arrangement prevents inadvertent movement of the upper jaw 18 from the closed condition to the open condition, though it should be understood that other locking arrangements (e.g., a magnetic interlock) may be employed without departing from the scope of the present disclosure.

The second carriage 16 is similarly configured to the first carriage 14, with an upper jaw 34 that may be configured in accordance with the foregoing description of the upper jaw 18 of the first carriage 14 and a lower jaw 36 that may be configured in accordance with the foregoing description of the lower jaw 20 of the first carriage 14.

As will be described in greater detail, the second carriage 16 is configured to move proximally and distally with respect to the first carriage 14 (and with respect to the housing 12), but in an initial or default position that is illustrated in Fig. 2, the second carriage 16 is positioned with a second portion 22b of the first dock 24 (which is defined by the lower jaw 36 of the second carriage 16) aligned with the first portion 22a of the first dock 24 and a second portion 26b of the second dock 28 (which is defined by the lower jaw 36 of the second carriage 16) aligned with the first portion 26a of the second dock 28. In this initial or default position, the first portion of each proximal slot is aligned with the second portion of the proximal slot of the same dock, while the first portion of each distal slot is aligned with the second portion of the distal slot of the same dock.

When using the sterile connection device 10 to execute a "single-pair" sterile connection procedure (as shown in Fig. 3), with the second carriage 16 in its initial or default position (and the upper jaws 18 and 34 in their open conditions), a proximal tube P may be inserted into the proximal slot of the first dock 24, with the proximal tube P being partially received by the first portion of the proximal slot of the first dock 24 (defined by the lower jaw 20 of the first carriage 14) and partially received by the second portion of the proximal slot of the first dock 24 (defined by the lower jaw 36 of the second carriage 16). Similarly, a distal tube D may be inserted into the distal slot of the first dock 24, with the distal tube D being partially received by the first portion of the distal slot of the first dock 24 (defined by the lower jaw 20 of the first carriage 14) and partially received by the second portion of the distal slot of the first dock (defined by the lower jaw 36 of the second carriage 16). Alternatively, rather than the pair of tubes P and D being received within the slots of the first dock 24, they may instead be received within the slots of the second dock 28 (with the same relative positioning described above with regard to an arrangement in which the tubes P and D are received by the slots of the first dock 24). The proximal and distal tubes P and D are mounted to the sterile connection device 10 in opposing orientations, with a (typically sealed) end of one of the tubes P, D positioned closer to the first carriage 14 and a (typically sealed) end of the other one of the tubes P, D positioned closer to the second carriage 16. To that end, the upper surface or face of the housing 12 may be provided with indicia to indicate the positions and orientations into which the tubes P and D are to be placed at the beginning of a "single-pair" sterile connection procedure.

When using the sterile connection device 10 to execute a "double-pair" sterile connection procedure (as shown in Fig. 2), with the second carriage 16 in its initial or default position (and the upper jaws 18 and 34 in their open conditions), a first proximal tube A is inserted into the proximal slot of the first dock 24, with the first proximal tube A being partially received by the first portion of the proximal slot of the first dock 24 and partially received by the second portion of the proximal slot of the first dock 24. A first distal tube B is inserted into the distal slot of the first dock 24, with the first distal tube B being partially received by the first portion of the distal slot of the first dock 24 and partially received by the second portion of the distal slot of the first dock. Similarly, a second proximal tube E is inserted into the proximal slot of the second dock 28 (with the second proximal tube E being partially received by the first portion of the proximal slot of the second dock 28 and partially received by the second portion of the proximal slot of the second dock 28) and a second distal tube F is inserted into the distal slot of the second dock 28 (with the second distal tube F being partially received by the first portion of the distal slot of the second dock 28 and partially received by the second portion of the distal slot of the second dock 28).

In addition to the carriages 14 and 16, the sterile connection device 10 also includes a blade handling assembly 40 (Figs. 3-5) incorporated into the housing 12. The blade handling assembly 40 may be differently configured without departing from the scope of the present disclosure, but in any case is configured to move a cutting blade 42 (Fig. 6) into different positions within the housing 12 during the course of a sterile connection procedure. In the illustrated embodiment, the blade handling assembly 40 includes a first linear drive motor 44 (Figs. 3-5), which is configured to enable movement in proximal and distal (longitudinal) directions. As will be described in greater detail, the first linear drive motor 44 is actuated to move a cutting blade 42 into position to be heated, along with optionally moving the second carriage 16 in proximal and distal directions. While a linear drive motor is illustrated, it should be understood that other mechanisms may be provided to effect proximal and distal movement of one or more components of the sterile connection device 10 (including a cutting blade 42) relative to the housing 12.

The illustrated blade handling assembly 40 includes a second linear drive motor 46 (Fig. 5), which is configured to enable movement in upward and downward directions within the housing 12 during the course of a sterile connection procedure. As will be described in greater detail, the second linear drive motor 46 is actuated to move the cutting blade 42 (after is has been heated) vertically into and out of a cutting position. While a linear drive motor is illustrated, it should be understood that other mechanisms may be provided to effect vertical movement of one or more components of the sterile connection device 10 (including a cutting blade 42) relative to the housing 12.

Regardless of the particular configuration of the blade handling assembly 40, it is configured to move a cutting blade from a dispensing position within the housing 12 to an intermediate position (where the cutting blade is heated) and then to a cutting position (to cut a pair of tubes received by one of the docks 24 and 28). The blade handling assembly 40 may move a cutting blade into additional positions (e.g., a disposal position, which will be described herein) without departing from the scope of the present disclosure.

In the illustrated embodiment, the dispensing position coincides with the position from which a cutting blade 42 is dispensed from of a blade cartridge 48 (Figs. 7-10). While the sterile connection device 10 is configured as a durable, reusable item, the cutting blades 42 (and, typically the blade cartridge 48) are configured as single-use, disposable items. In one embodiment, the cutting blades 42 are configured as flat or planar wafers formed of a single material or blend of materials (e.g., solid metal, such as copper), in contrast to the multi-layer cutting elements of other sterile connection devices. However, it is within the scope of the present disclosure for the cutting blades to be differently configured (e.g., as multi-layer cutting elements having a resistive circuit layer) without departing form the scope of the present disclosure. The cutting blade 42 of Fig. 6 is shown as being substantially rectangular, but it should be understood that the shape of the cutting blade 42 may vary without departing from the scope of the present disclosure.

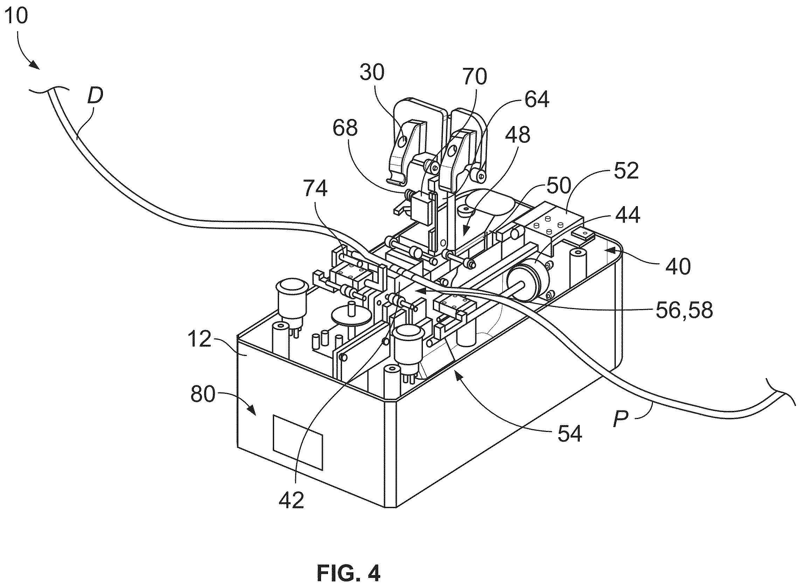

The illustrated housing 12 defines a slot or cavity configured to at least partially receive a blade cartridge 48. One end 50 of the blade cartridge 48 includes an opening from which the cutting blades 42 are individually dispensed. As best shown in Fig. 4, the open end 50 of the blade cartridge 48 is oriented adjacent to the first linear drive motor 44 of the blade handling assembly 40. The first linear drive motor 44 is driven in a first or forward direction to move a pusher 52 in a proximal direction so as to contact a cutting blade 42 positioned at the open end 50 of the blade cartridge 48 and move the cutting blade 42 proximally and directly to an intermediate position between and beneath the first and second carriages 14 and 16. While Fig. 4 illustrates the blade cartridge 48 as being positioned distally of the carriages 14 and 16, with a cutting blade 42 being moved in a proximal direction into an intermediate position between and beneath the carriages 14 and 16, it should be understood that the blade cartridge 48 may instead be positioned proximally of the carriages 14 and 16, with the blade handling assembly 40 moving a cutting blade 42 in a distal direction into an intermediate position between and beneath the carriages 14 and 16.

The illustrated blade cartridge 48 is spring-loaded, such that dispensing one cutting blade 42 from the open end 50 will cause one or more springs disposed within the blade cartridge 48 to press a subsequent cutting blade 42 into position at the open end 50, where it is ready to be dispensed. As will be described in greater detail herein, a single cutting blade 42 is dispensed after a "single-pair" sterile connection procedure has been completed, with two cutting blades 42 being dispensed after a "double-pair" sterile connection procedure has been completed. Once the blade cartridge 48 has been emptied (which may be determined by a sensor of the sterile connection device 10, for example), it may be removed and replaced with a fully loaded blade cartridge 48. Alternatively, the blade cartridge 48 may be removed, refilled with cutting blades 42, and then mounted back into the housing 12.

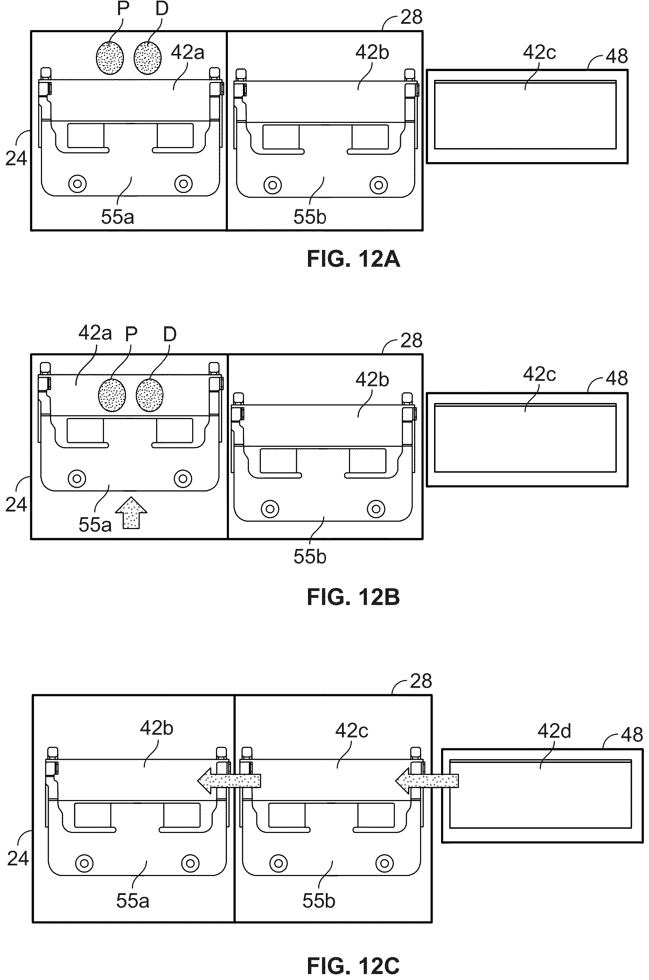

In the intermediate position, a cutting blade 42 is received and heated by a blade heating assembly 54 incorporated into the housing 12. Each cutting blade 42 is sized to cut only one pair of tubes (either a pair of tubes received by the first dock 24 or a pair of tubes received by the second dock 28), so the blade heating assembly 54 includes a first holder 55a associated with the first dock 24 and a second holder 55b associated with the second dock 28 (Figs. 12A-13D), with each holder 55a, 55b being configured to receive a single cutting blade 42. The first holder 55a is positioned between and beneath the two portions 22a and 22b of the first dock 24, while the second holder 55b is positioned between and beneath the two portions 26a and 26b of the second dock 28. In the illustrated embodiment, the two docks 24 and 28 are provided with separate holders 55a and 55b, which allows for the cutting blade 42 associated with one of the docks to be moved upwardly toward that dock into a cutting position without the cutting blade 42 associated with the other dock also being moved upwardly toward that dock. In another embodiment, the two separate holders 55a and 55b are replaced by a single, elongated holder configured to receive two cutting blades 42 (one aligned with the first dock 24 and the other aligned with the second dock 28) and positioned between and beneath the two portions of the first and second docks 24 and 28.

In addition to the holders 55a and 55b, the blade heating assembly 54 includes a heating element 56 and a thermocouple 58 (which are both shown in Fig. 4), along with a temperature controller 60 (Fig. 3). In the illustrated embodiment a single blade heating assembly 54 is associated with both of the holders 55a and 55b, though it is within the scope of the present disclosure for each holder 55a, 55b to include a dedicated blade heating assembly (each with its own heating element, thermocouple, and temperature controller). The heating element 56 may be variously configured without departing from the scope of the present disclosure, provided that it is suitable for applying heat to the cutting blades 42 held by one or both of the holders 55a, 55b prior to the cutting blade(s) 42 (and, optionally, the heating element 56 as well) being moved from the intermediate position to the cutting position (e.g., in an upward direction by the second linear drive motor 46). In an exemplary embodiment, the heating element 56 is configured as a ceramic heater having one or more plates that each contact or are positioned directly adjacent to at least a portion of the cutting blade(s) 42 to heat at least a portion of the cutting blade(s) 42 by conductive heating (as opposed to resistive heating, which is employed when a cutting element includes a resistive circuit layer). In the illustrated embodiment, the upper edge of a cutting blade 42 is brought into contact with the pair of tubes received by the associated dock to cut the tubes, such that it is advantageous for the heating element 56 to be configured to apply heat to at least part of the upper edge of the cutting blade 42. However, as will be described in greater detail, the cut end of one of the tubes of each pair of tubes is moved along the adjacent face of the associated cutting blade 42 prior to joining the cut ends, so it may be advantageous for a larger portion of the cutting blade 42 (which may include substantially the entire cutting blade 42) to be heated.

The manner in which the heating element 56 is itself heated depends upon the particular configuration of the blade heating assembly 54. For example, in the exemplary embodiment in which the heating element 56 is configured as a ceramic heater, the one or more ceramic plates of the heating element 56 are heated by application of electricity to the plate(s) by the temperature controller 60. The temperature controller 60 may be variously configured without departing from the scope of the present disclosure, with the temperature controller 60 being of the type marketed as the E5DC temperature controller by Omron Corporation of Kyoto, Japan in an exemplary embodiment.

The illustrated heating element 56 includes an internal thermocouple 58 that is electrically coupled to the temperature controller 60. The thermocouple 58 produces a voltage that is dependent upon its temperature (which is dependent upon the temperature of the associated heating element 56), with the voltage being delivered to the temperature controller 60. Upon the temperature controller 60 receiving a voltage that is indicative of a target temperature (which is approximately 300° C in an exemplary embodiment), the temperature controller 60 transmits a signal to a system controller 62 (Figs. 3 and 5). The system controller 62 uses the signal from the temperature controller 60 to determine when the cutting blades 42 have been sufficiently heated. This may include the system controller 62 determining that the cutting blades 42 have been sufficiently heated upon receiving the signal from the temperature controller 60 or the system controller 62 making that determination at some later time (e.g., after a predetermined amount of time). Notably, in this embodiment, the temperature of the cutting blade 42 itself is not monitored, but rather only the temperature of the heating element 56. In other embodiments, the temperature of the cutting blade 42 itself may be monitored, though monitoring only the temperature of the heating element 56 may be sufficient to determine the temperature of the cutting blade 42, particularly when a simple cutting blade (e.g., a solid copper blade) is employed, on account of the cutting blade 42 being heated to a predictable temperature upon application of a particular level of heat for a particular amount of time.

As for the system controller 62, it may be variously configured without departing from the scope of the present disclosure, provided that it is configured to coordinate the various tasks carried out by the components of the sterile connection device 10 during a sterile connection procedure. In one embodiment, the system controller 62 may include a microprocessor (which, in fact may include multiple physical and/or virtual processors). According to other embodiments, the system controller 62 may include one or more electrical circuits designed to carry out the actions described herein. In fact, the system controller 62 may include a microprocessor and other circuits or circuitry. In addition, the system controller 62 may include one or more memories. The instructions by which the microprocessor is programmed may be stored on the memory associated with the microprocessor, which memory/memories may include one or more tangible non-transitory computer readable memories, having computer executable instructions stored thereon, which when executed by the microprocessor, may cause the microprocessor to carry out one or more actions as described herein.

Turning now to an exemplary "single-pair" sterile connection procedure, with the upper jaws 18 and 34 in their open condition (as in Fig. 3) an operator places two tubes P and D into the proximal and distal slots of one of the docks 24, 28 defined by the lower jaws 20 and 36 of the carriages 14 and 16. The (typically sealed) end of the proximal tube P is positioned adjacent to the first carriage 14, while the (typically sealed) end of the distal tube D is positioned adjacent to the second carriage 16, as shown in Fig. 3. Notably, the tubes P and D may have different outer and inner diameters (within an allowable range), rather than necessarily having the same outer and inner diameters.

With the tubes P and D in place, the operator moves the upper jaws 18 and 34 from their open condition to their closed condition. The upper jaws 18 and 34 may be independently movable between their open and closed conditions or may be configured to move together from the open condition to the closed condition and/or from the closed condition to the open condition. As described above, in their closed conditions, the upper jaws 18 and 34 cover the lower jaws 20 and 36 and secure the tubes P and D within the carriages 14 and 16.

In the illustrated embodiment, a tubing clamp 64 is configured to contact and compress the tubes P and D at a position between the first and second portions of the dock 24, 28 in which they are received when the upper jaws 18 and 34 are in the closed condition. By compressing the tubes P and D, the tubing clamp 64 moves any liquid away from the site at which the tubing clamp 64 engages the tubes P and D. As will be described in greater detail, this site corresponds to the location at which the tubes P and D are cut by the heated cutting blade 42, such that the tubing clamp 64 serves to clear liquid from the location at which the tubes P and D are cut. By clearing liquid from this location, it becomes possible to connect and join two tubes when at least one of them contains a liquid. Thus, the inclusion of a tubing clamp 64 allows for a "dry-to-dry" connection (in which neither tube P, D contains a liquid), a "dry-to-wet" connection (in which one tube P, D contains a liquid), and a "wet-to-wet" connection (in which both tubes P and D contain a liquid).

The tubing clamp 64 may be variously configured without departing from the scope of the present disclosure. In one embodiment, the tubing clamp 64 is associated with one or both of the upper jaws 18 and 34, such that movement of the associated upper jaw(s) 18, 34 between open and closed conditions will cause similar movement of the tubing clamp 64. In such a configuration, moving the upper jaws 18 and 34 to their closed condition will move the tubing clamp 64 into contact with the tubes P and D. The tubing clamp 64 may be formed of a metallic material or some other generally rigid material (to ensure that the tubing clamp 64 will compress the tubes P and D when the tubing clamp 64 is moved into contact with the tubes P and D), with the tubing clamp 64 being spring-loaded to control the amount of force applied to the tubes P and D by the tubing clamp 64.

With the upper jaws 18 and 34 in their closed condition, the operator presses a "start" button 66 to continue the sterile connection procedure. In the illustrated embodiment, in which a single "start" button 66 is provided, the system controller 62 may be programmed to determine whether to execute a "single-pair" procedure or a "double-pair" procedure upon the "start" button 66 being pressed. This may include one or more of the portions of the first and second docks 24 and 28 including a sensor 67 configured to detect whether a pair of tubes is received within the associated dock 24, 28. When the sensors 67 transmit signals to the system controller 62 indicating that only one pair of tubes has been properly mounted to the sterile connection device 10 (further indicating the dock 24, 28 into which the pair of tubes has been mounted), the system controller 62 proceeds to execute a "single-pair" sterile connection procedure. On the other hand, when the sensors 67 transmit signals to the system controller 62 indicating that two pairs of tubes have been properly mounted to the sterile connection device 10, the system controller 62 proceeds to execute a "double-pair" sterile connection procedure. In one embodiment, the system controller 62 may decline to execute a sterile connection procedure when the signals from the sensors 67 indicate that a pair of tubes has not been properly mounted to the sterile connection device 10 (e.g., if one tube is placed into the first dock 24 and one other tube is placed into the second dock 28).

In the illustrated embodiment, a single "start" button 66 is provided, but in another embodiment, the sterile connection device 10 may be provided with a first "start" button that is pressed when a "single-pair" sterile connection procedure is to be executed by the system controller 62 and a second "start" button that is pressed when a "double-pair" sterile connection procedure is to be executed by the system controller 62. In such an embodiment, pressing the "start" button designated for a "single-pair" sterile connection procedure may include sending a "single blade replacement" input to the system controller 62, which instructs the system controller 62 that a single cutting blade 42 is to be replaced after the procedure has been completed (as will be described in greater detail herein). Similarly, pressing the "start" button designated for a "double-pair" sterile connection procedure may include sending a "double blade replacement" input to the system controller 62, which instructs the system controller 62 that two cutting blades 42 are to be replaced after the procedure has been completed (as will be described in greater detail herein). In one embodiment, the system controller 62 may be programmed to require receipt of the "single blade replacement" input prior to executing a "single-pair" sterile connection procedure and to require receipt of the "double blade replacement" input prior to executing a "double-pair" sterile connection procedure.

Typically, cutting blades 42 are already positioned at the intermediate position (received by the holders 55a and 55b and ready to be heated) when the "start" button 66 is pressed. In this case, pressing the "start" button 66 causes the system controller 62 to command the blade heating assembly 54 to heat the cutting blades 42 to a desired temperature (which may be estimated based on the temperature of the heating element 56 of the blade heating assembly 54, as explained above). If a pair of cutting blades 42 is not present in the intermediate position (as determined by a sensor, for example), pressing the "start" button 66 causes the system controller 62 to command the blade handling assembly 40 to move the appropriate number of cutting blades 42 from the blade cartridge 48 to the intermediate position (e.g., by action of the first linear drive motor 44), followed by the system controller 62 commanding the blade heating assembly 54 to heat the cutting blades 42 to a target temperature. In an alternative embodiment, rather than heating both of the cutting blades 42 that are present in the holders 55a and 55b, the system controller 62 may be programmed to control the blade heating assembly 54 to heat only one of the cutting blades 42 (namely, the one aligned with the dock 24, 28 in which the tubes P and D are received) and not the other cutting blade 42.

Upon the cutting blade 42 that is aligned with the tubes P and D reaching the target temperature (e.g., as determined by the system controller 62, based upon a signal received from the temperature controller 60), the heated cutting blade 42 is moved from the intermediate position to the cutting position. In the illustrated embodiment, this movement is in the upward direction and carried out by actuation of the second linear drive motor 46 of the blade handling assembly 40. As described above, this may include only the cutting blade 42 aligned with the tubes P and D being moved from the intermediate position to the cutting position or both of the cutting blades 42 positioned within the holders 55a and 55b being moved to the cutting position. The heated cutting blade 42 aligned with the tubes P and D presses against the tubes P and D, which are in turn pressed against the tubing clamp 64. The tubing clamp 64 may remain in position while the heated cutting blade 42 presses against the tubes P and D or may be moved by the heated cutting blade 42 (and/or the holders 55a and/or 55b). In one embodiment, the tubing clamp 64 is moved from its lowered position (which corresponds to the closed condition of the upper jaws 18 and 34) to a raised position by the heated cutting blade 42 and/or the holders 55a and/or 55b as the cutting blade 42 cuts the tubes P and D, with the tubing clamp 64 being locked into the raised position by a spring 68 moving a latching mechanism 70 (Fig. 4) into place, or the like.

When the cutting blade 42 has cut through the tubes P and D, the second carriage 16 is moved distally with respect to the first carriage 14 and with respect to the cutting blade 42 (which remains in its cutting position). This movement draws the cut end of the proximal tube P positioned within the second portion of the proximal slot of one of the docks 24, 28 along the heated cutting blade 42, into alignment with the cut end of the distal tube D positioned within the first portion of the distal slot of that dock 24, 28. The second carriage 16 may be moved by any suitable mechanism, with the second carriage 16 being moved in the distal direction by the first linear drive motor 44 as the first linear drive motor 44 moves distally into position to deliver a subsequent cutting blade 42 to the heating element 56, in an exemplary embodiment. A spring 72 (Fig. 5) may also (or alternatively) be employed to cause this distal movement of the second carriage 16.

With the cut ends of the tubes P and D so aligned, the system controller 62 commands the blade handling assembly 40 to move the cutting blade(s) 42 back to the intermediate position (e.g., by actuating the second linear drive motor 46 in reverse to lower the heated cutting blade(s) 42) and then commands the first carriage 14 to be moved laterally toward the second carriage 16 so as to bring the cut ends of the tubes P and D into contact with each other. This movement may be carried out by any suitable mechanism, such as (for example) a spring 74 (Fig. 4) pressing the first carriage 16 toward the second carriage 16 or a third linear drive motor (not illustrated) moving the first carriage 14 toward the second carriage 16.

The cut ends of the tubes P and D are pressed together for a predetermined amount of time to create a joint and cool, after which time the system controller 62 advances to the next stage of the procedure. In this stage, the operator is notified that the tubes P and D have been sterilely connected to define a joined tube J (Fig. 5). This notification may be provided in the form of an audible alert (e.g., an alarm) and/or a visual alert (e.g., a flashing light or an icon shown on a screen), for example. At this time, the system controller 62 unlocks the upper jaws 18 and 34 (if they are locked into their closed condition), which allows the operator to move the upper jaws 18 and 34 back into their open condition and then remove the joined tube J from the lower jaws 20 and 36 of the carriages 14 and 16 (along with the cut-off ends of the tubes P and D, which may be discarded). If required, the operator may manipulate the joined tube to ensure that the joint is secure and open for fluid flow (e.g., by pinching the joint).

In one embodiment, at the end of the procedure, the operator presses a "reset" button 76 to reset the sterile connection device 10. If pressing the "start" button at the beginning of the procedure does not include a "single blade replacement" input being sent to the system controller 62, pressing the "reset" button 76 may instead cause a "single blade replacement" input to be sent to the system controller 62. The "single blade replacement" input instructs the system controller 62 to control the blade handling assembly 40 to move a single cutting blade 42 from the blade cartridge 48 into one of the holders in the intermediate position, which presses the heated cutting blade 42 that was used in the just-completed procedure out of the holder and into a disposal receptacle 78 (Fig. 5). The disposal receptacle 78 may be differently configured without departing from the scope of the present disclosure, with the illustrated disposal receptacle 78 being configured as a drawer positioned at the front or proximal face of the housing 12 and including a door or access 80 that may be opened to allow spent cutting blades 42 to be removed from the disposal receptacle 78.

In the illustrated embodiment, a single "reset" button 76 is provided, but in another embodiment, the sterile connection device 10 may be provided with a first "reset" button that is pressed when a "single-pair" sterile connection procedure has been completed and a second "reset" button that is pressed when a "double-pair" sterile connection procedure has been completed. In such an embodiment, pressing the "reset" button designated for resetting the sterile connection device 10 after a "single-pair" sterile connection procedure may include sending a "single blade replacement" input to the system controller 62, as described above. Similarly, pressing the "reset" button designated for resetting the sterile connection device 10 after a "double-pair" sterile connection procedure may include sending a "double blade replacement" input to the system controller 62, which instructs the system controller 62 that two cutting blades 42 are to be replaced in the course of resetting the sterile connection device 10 (as will be described in greater detail herein). If only a single "reset" button 76 is provided (as in the illustrated embodiment), the system controller 62 may be programmed to determine whether a "single-pair" sterile connection procedure or a "double-pair" sterile connection procedure has just been completed and replace the appropriate number of used cutting blades 42 when resetting the sterile connection device 10. In one embodiment, the system controller 62 may be programmed to request entry of either the "single blade replacement" input or the "double blade replacement" input at the end of a sterile connection procedure, prior to executing a subsequent sterile connection procedure. This may include, for example, the system controller 62 causing a light associated with the "reset" button(s) to flash at the end of a procedure, alerting the operator that a "reset" button must be pressed (in order to deliver the requested blade replacement input to the system controller 62) before a subsequent sterile connection procedure may be executed.

In addition to replacing one or more of the cutting blades 42, the system controller 62 also commands the first and second carriages 14 and 16 to return to their default or initial positions (namely, by moving the first carriage 14 laterally away from the second carriage 16, while moving the second carriage 16 in a proximal direction). In one embodiment, the first linear drive motor 42 is responsible for simultaneously moving the second carriage 16 to its original position and moving a cutting blade 42 from the blade cartridge 48 to the intermediate position, though it is within the scope of the present disclosure for different mechanisms to be employed.

Figs. 2 and 9-11 illustrate use of the sterile connection device 10 for executing an exemplary "double-pair" sterile connection procedure. As shown in Fig. 2, a first tube A is inserted into the proximal slot of the first dock 24, with the first tube A being partially received by the first portion of the proximal slot of the first dock 24 (defined by the lower jaw 20 of the first carriage 14) and partially received by the second portion of the proximal slot of the first dock 24 (defined by the lower jaw 36 of the second carriage 16). Similarly, a second tube B is inserted into the distal slot of the first dock 24, with a third tube E inserted into the proximal slot of the second dock 28 and a fourth tube F inserted into the distal slot of the second dock 28. The first and second tubes A and B (which are to be joined together) are mounted to the sterile connection device 10 in opposing orientations, with a (typically sealed) end of one of the tubes A, B positioned closer to the first carriage 14 and a (typically sealed) end of the other one of the tubes A, B positioned closer to the second carriage 16. The third and fourth tubes E and F (which are to be joined together) are similarly mounted to the sterile connection device 10 in opposing orientations, as can be seen in Fig. 2.

With the tubes in place, the operator moves the respective upper jaws 18 and 34 of the first and second carriages 14 and 16 from their open condition (Fig. 2) to their closed condition (Fig. 9) to secure the tubes within the carriages 14 and 16. If provided (as in the illustrated embodiment), a tubing clamp 64 contacts and compresses the tubes at a position between the first and second portions of each dock when the upper jaws 18 and 34 are in the closed condition to move any liquid away from the location at which the tubes are to be cut.

With the upper jaws 18 and 34 in their closed condition, the operator presses the "start" button 66 to continue the sterile connection procedure. As described above, this may include the operator pressing the only available "start" button 66 (with the system controller 62 determining that a "double-pair" sterile connection procedure is to be executed) or the operator pressing a "start" button specifically designated for beginning a "double-pair" sterile connection procedure. As described above, a pair of cutting blades 42 will typically be positioned at an intermediate position (received by the holders 55a and 55b and ready to be heated) when a "start" button is pressed. In this case, pressing the "start" button causes the system controller 62 to command the blade heating assembly 54 to heat the two cutting blades 42 to a desired temperature. If both cutting blades 42 are not present in the intermediate position (as determined by a sensor, for example), pressing the "start" button causes the system controller 62 to command the blade handling assembly 40 to move one or two cutting blades 42 from the blade cartridge 48 to the intermediate position, followed by the system controller 62 commanding the blade heating assembly 54 to heat the cutting blades 42 to a target temperature.

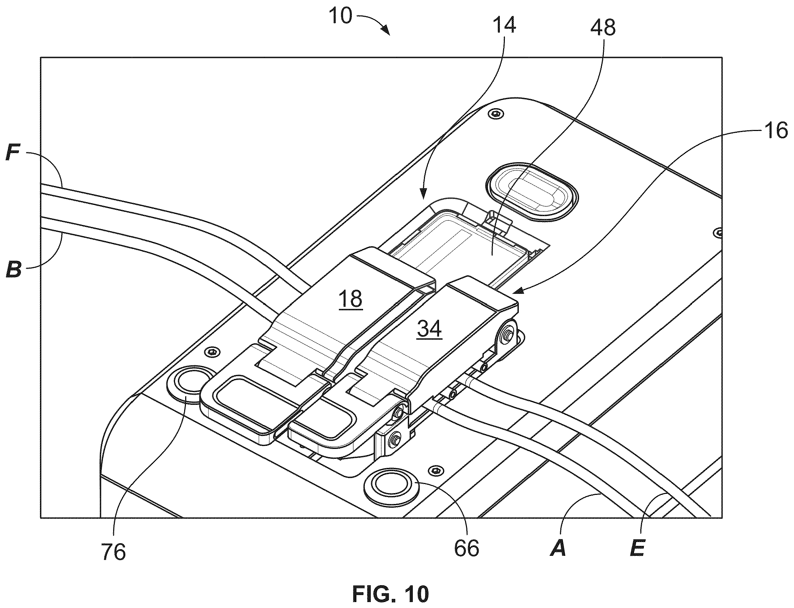

Upon the cutting blades 42 reaching the target temperature, the heated cutting blades 42 moved from the intermediate position to the cutting position by the blade handling assembly 40. The heated cutting blades 42 press against the tubes, which are in turn pressed against the tubing clamp 64 to cut through the tubes. When the cutting blades 42 have cut through the tubes, the second carriage 16 is moved distally with respect to the first carriage 14 and with respect to the cutting blades 42 (which remains in their cutting position). This movement draws the cut ends of the first tube A and the third tube E along the heated cutting blades 42, into alignment with the cut ends of the second tube B and the fourth tube F, respectively (Fig. 10).

With the cut ends of the tubes so aligned, the system controller 62 commands the blade handling assembly 40 to move the cutting blades 42 back to the intermediate position and then commands the first carriage 14 to be moved laterally toward the second carriage 16 so as to bring the aligned cut ends of the two pairs of tubes into contact with each other. The cut ends of the first and second tubes A and B and of the third and fourth tubes E and F are pressed together for a predetermined amount of time to create a joint and cool, after which time the system controller 62 advances to the next stage of the procedure.

In the next stage, the operator is notified that the first and second tubes A and B have been sterilely connected to define a first joined tube C, with the third and fourth tube E and F being sterilely connected to define a second joined tube G. At this time, the system controller 62 unlocks the upper jaws 18 and 34 (if they are locked into their closed condition), which allows the operator to move the upper jaws 18 and 34 back into their open condition (Fig. 11) and then remove the joined tubes C and G from the lower jaws 20 and 36 of the carriages 14 and 16 (along with the cut-off ends of the tubes, which may be discarded). If required, the operator may manipulate the joined tubes to ensure that the joints are secure and open for fluid flow (e.g., by pinching the joint). In one embodiment, at the end of the procedure, the operator presses a "reset" button 76 to reset the sterile connection device 10, which includes sending a "double blade replacement" input to the system controller 62 (instructing the system controller 62 to control the blade handling assembly 40 to replace the two used cutting blades 42 when resetting the device).

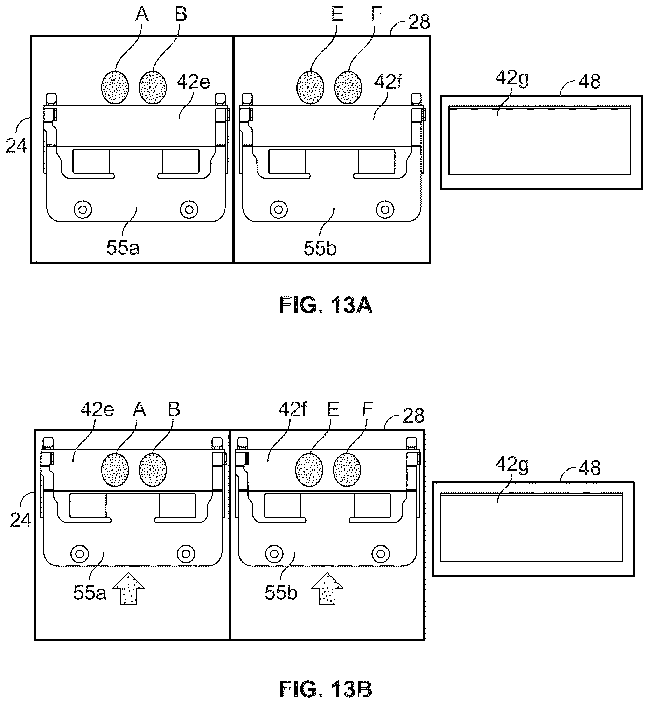

Turning now more particularly to the manner in which one or more cutting blades are replaced after completion of a sterile connection procedure, Figs. 12A-12C illustrate an exemplary "single-pair" sterile connection procedure in which a single cutting blade is replaced after the tubes have been joined and Figs. 13A-13C illustrate an exemplary "double-pair" sterile connection procedure in which two cutting blades are replaced after the tubes have been joined.

In the exemplary "single-pair" sterile connection procedure illustrated in Figs. 12A-12C, a single pair of tubes P and D is mounted to the first dock 24, with no tubes being mounted to the second dock 28. A first cutting blade 42a is retained in the holder 55a associated with the first dock 24, a second cutting blade 42b is retained in the holder 55b associated with the second dock 24, and a replacement cutting blade 42c is positioned at the open end 50 of the blade cartridge 48.

With the cutting blades 42a and 42b in their intermediate positions (Fig. 12A), the cutting blade 42a associated with the first dock 24 is heated to the target temperature. The cutting blade 42b associated with the second dock 28 may also be heated or may instead be unheated. Once the cutting blade 42a associated with the first dock 24 has reached the target temperature, it is moved from the intermediate position into its cutting position to cut the tubes P and D, as shown in Fig. 12B. The cutting blade 42b associated with the second dock 28 may also be moved from the intermediate position into its cutting position or (as shown in Fig. 12B) it may instead remain in its intermediate position. After the tubes P and D have been cut, they are moved into alignment and the cutting blade 42a associated with the first dock 24 is moved from its cutting position back to its intermediate position to allow for the tubes P and D to be joined.

When the joined tubes have been removed from the first dock 24, the system controller 62 controls the blade handling assembly 40 to dispense the replacement cutting blade 42c from the blade cartridge 48. The blade handling assembly 40 moves the replacement cutting blade 42c in a longitudinal direction (which is a proximal direction, in the illustrated embodiment), which advances the replacement cutting blade 42c toward the holder 55b associated with the second dock 28. This movement causes the replacement cutting blade 42c to contact the unused cutting blade 42b positioned in the second holder 55b and move that unused cutting blade 42b out of the second holder 55b and toward the first holder 55a. The movement of the unused cutting blade 42b into the first holder 55a causes the unused cutting blade 42b to contact the used cutting blade 42a positioned in the first holder 55a and move that used cutting blade 42a out of the first holder 55a. The blade handling assembly 40 continues advancing the replacement cutting blade 42c in the longitudinal (proximal) direction until the replacement cutting blade 42c is properly positioned within the second holder 55b, the unused cutting blade 42b has been moved entirely out of association with the second dock 28 and into the holder 55a associated with the first dock 24, and the used cutting blade 42a has been moved entirely out of association with the first dock 24 (and into a disposal receptacle 78, for example), with a new replacement cutting blade 42d being advanced to the open end 50 of the blade cartridge 48 (Fig. 12C). With the two unused cutting blades 42b and 42c in the intermediate positions, a subsequent sterile connection procedure (which could be either a "single-pair" sterile connection procedure or a "double-pair" sterile connection procedure) may be executed.

While Figs. 12A-12C illustrate a "single-pair" sterile connection procedure in which a pair of tubes mounted within the first dock 24 is joined, it should be understood that the same principle may be applied to a "single-pair" sterile connection procedure executed for a pair of tubes mounted within the second dock 28. In that case, the replacement cutting blade would be dispensed in a distal longitudinal direction (from a blade cartridge 48 positioned proximally of the first dock 24) to move the replacement blade into the holder 55a associated with the first dock 24, thus advancing the unused cutting blade from the first holder 55a into the second holder 55b (in association with the second dock 28) and advancing the used cutting blade out of the second holder 55b and out of association with the second dock 28.

In the exemplary "double-pair" sterile connection procedure illustrated in Figs. 13A-13D, a first pair of tubes A and B is mounted to the first dock 24, with a second pair of tubes E and F being mounted to the second dock 28. A first cutting blade 42e is retained in the holder 55a associated with the first dock 24, a second cutting blade 42f is retained in the holder 55b associated with the second dock 24, and a replacement cutting blade 42g is positioned at the open end 50 of the blade cartridge 48.

With the cutting blades 42e and 42f in their intermediate positions (Fig. 13A), both cutting blades 42e and 42f are heated to the target temperature. Once the cutting blades 42e and 42f have reached the target temperature, they are moved from the intermediate position into their cutting position, with the heated cutting blade 42e in the first holder 55a cutting the first pair of tubes A and B and the heated cutting blade 42f in the second holder 55b cutting the second pair of tubes E and F, as shown in Fig. 13B. After all four tubes have been simultaneously cut, they are moved into alignment (with tubes A and B aligned and with tubes E and F aligned) and the cutting blades 42e and 42f are moved from their cutting position back to their intermediate position to allow for the two pairs of tubes to be simultaneously joined.

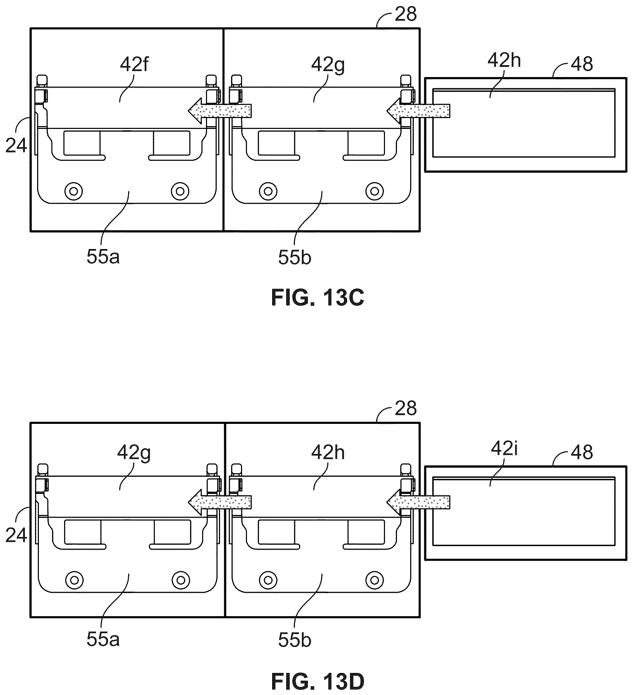

When the two joined tubes have been removed from the docks 24 and 28, the system controller 62 controls the blade handling assembly 40 to dispense the replacement cutting blade 42g from the blade cartridge 48. The blade handling assembly 40 moves the replacement cutting blade 42g in a longitudinal direction (which is a proximal direction, in the illustrated embodiment), which advances the replacement cutting blade 42g toward the holder 55b associated with the second dock 28. This movement causes the replacement cutting blade 42g to contact the used cutting blade 42f positioned in the second holder 55b and move that used cutting blade 42f out of the second holder 55b and toward the first holder 55a. The movement of the used cutting blade 42f into the first holder 55a causes the used cutting blade 42f to contact the used cutting blade 42e positioned in the first holder 55a and move that used cutting blade 42e out of the first holder 55a. The blade handling assembly 40 continues advancing the replacement cutting blade 42g in the longitudinal (proximal) direction until the replacement cutting blade 42g is properly positioned within the second holder 55b, the used cutting blade 42f has been moved entirely out of association with the second dock 28 and into the holder 55a associated with the first dock 24, and the used cutting blade 42g has been moved entirely out of association with the first dock 24 (and into a disposal receptacle 78, for example), with a second replacement cutting blade 42h being advanced to the open end 50 of the blade cartridge 48 (Fig. 13C).

Next, the blade handling assembly 40 moves the second replacement cutting blade 42h in the same longitudinal direction as the first replacement cutting blade 42g was moved (which is a proximal direction, in the illustrated embodiment), which advances the second replacement cutting blade 42h toward the holder 55b associated with the second dock 28. This movement causes the second replacement cutting blade 42h to contact the first replacement cutting blade 42g positioned in the second holder 55b and move that replacement cutting blade 42g out of the second holder 55b and toward the first holder 55a. The movement of the first replacement cutting blade 42g into the first holder 55a causes the first replacement cutting blade 42g to contact the used cutting blade 42f positioned in the first holder 55a and move that used cutting blade 42f out of the first holder 55a. The blade handling assembly 40 continues advancing the second replacement cutting blade 42h in the longitudinal (proximal) direction until the second replacement cutting blade 42h is properly positioned within the second holder 55b, the first replacement cutting blade 42g has been moved entirely out of association with the second dock 28 and into the holder 55a associated with the first dock 24, and the used cutting blade 42f has been moved entirely out of association with the first dock 24 (and into a disposal receptacle 78, for example), with a third replacement cutting blade 42i being advanced to the open end 50 of the blade cartridge 48 (Fig. 13D). With the two replacement cutting blades 42g and 42h in the intermediate positions, a subsequent sterile connection procedure (which could be either a "single-pair" sterile connection procedure or a "double-pair" sterile connection procedure) may be executed.

While Figs. 13A-13D illustrate a "double-pair" sterile connection procedure in which two replacement cutting blades are moved in a proximal longitudinal direction to replace a pair of used cutting blades, it should be understood that the replacement cutting blades may instead be moved in a distal longitudinal direction to replace the two used cutting blades. In that case, a first replacement cutting blade would be dispensed in a distal longitudinal direction (from a blade cartridge 48 positioned proximally of the first dock 24) to move the first replacement blade into the holder 55a associated with the first dock 24, thus advancing the first used cutting blade from the first holder 55a into the second holder 55b (in association with the second dock 28) and advancing the second used cutting blade out of the second holder 55b and out of association with the second dock 28. A second replacement cutting blade would then be dispensed from the blade cartridge 48 in the same longitudinal direction to move the second replacement cutting blade into the holder 55a associated with the first dock 24, thus advancing the first replacement cutting blade from the first holder 55a into the second holder 55b and advancing the first used cutting blade out of the second holder 55b and out of association with the second dock 28.

By serially dispensing cutting blades in this manner, it will be seen that a single sterile connection device may be used to simultaneously join two pairs of tubes or a single pair of tubes without requiring the use of "two-weld" blades (configured to simultaneously cut the ends off of two pairs of tubes). By using only "one-weld" cutting blades (configured to cut the ends off of only a single pair of tubes), the waste produced and the material cost of executing a "single-pair" sterile connection procedure is reduced compared to the same procedure being executing using a "two-weld" blade. Additionally, by using only "one-weld" cutting blades, the controls and components required to dispense the cutting blades may be less complicated and expensive than the controls and components that would be required if the sterile connection device were instead configured to selectively employ both "one-weld" blades and "two-weld" blades.

It is again emphasized that the illustrated sterile connection device 10 is merely exemplary and that sterile connection devices according to the present disclosure may be differently configured without departing from the scope of the present disclosure. This may include a sterile connection device having its components differently arranged (e.g., with a blade cartridge positioned proximally of the carriages instead of distally of the carriages) and/or a sterile connection device including additional components (e.g., a cord for connection to an external power source, a variety of sensors, and/or a touchscreen for use by an operator).

### Aspects

Aspect 1. A sterile connection device comprising: a housing; a first carriage including a first lower jaw defining a first portion of a first dock and a first portion of a second dock, and a first upper jaw configured to move between an open condition spaced away from the first lower jaw and a closed condition positioned adjacent to the first lower jaw; a second carriage positioned laterally of the first carriage and including a second lower jaw defining a second portion of the first dock and a second portion of the second dock, and a second upper jaw configured to move between an open condition spaced away from the second lower jaw and a closed condition positioned adjacent to the second lower jaw; a blade handling assembly configured to move cutting blades into a plurality of positions within the housing; a blade heating assembly; and a system controller programmed to execute a single-pair sterile connection procedure when a single pair of tubes is received by the first dock, no tubes are received by the second dock, a first cutting blade is associated with the first dock, a second cutting blade is associated with the second dock, and the first and second upper jaws are in the closed conditions and to execute a double-pair sterile connection procedure when a first pair of tubes is received by the first dock, a second pair of tubes is received by the second dock, the first cutting blade is associated with the first dock, the second cutting blade is associated with the second dock, and the first and second upper jaws are in the closed conditions, wherein the single-pair sterile connection procedure includes controlling the blade handling assembly, the blade heating assembly, and at least one of the first and second carriages to cut the single pair of tubes using the first cutting blade and then join the single pair of tubes, and controlling the blade handling assembly to advance a single replacement cutting blade in a longitudinal direction into association with the second dock, thereby advancing the second cutting blade in the longitudinal direction out of association with the second dock and into association with the first dock and advancing the first cutting blade in the longitudinal direction out of association with the first dock, and the double-pair sterile connection procedure includes controlling the blade handling assembly, the blade heating assembly, and at least one of the first and second carriages to cut the first pair of tubes using the first cutting blade and then join the first pair of tubes and to cut the second pair of tubes using the second cutting blade and then join the second pair of tubes, and controlling the blade handling assembly to advance a first replacement cutting blades in a longitudinal direction so as to place the first replacement cutting blade into association with the second dock, thereby advancing the second cutting blade in the longitudinal direction out of association with the second dock and into association with the first dock and advancing the first cutting blade in the longitudinal direction out of association with the first dock, and controlling the blade handling assembly to advance a second replacement cutting blade in the longitudinal direction so as to place the second replacement cutting blade into association with the second dock, thereby advancing the first replacement cutting blade in the longitudinal direction out of association with the second dock and into association with the first dock and advancing the second cutting blade in the longitudinal direction out of association with the first dock.

Aspect 2. The sterile connection device of Aspect 1, wherein the system controller is programmed to control the blade heating assembly to apply heat to the first cutting blade and not to the second cutting blade during the single-pair sterile connection procedure.

Aspect 3. The sterile connection device of Aspect 1, wherein the system controller is programmed to control the blade heating assembly to apply heat to the first cutting blade and to the second cutting blade during the single-pair sterile connection procedure.

Aspect 4. The sterile connection device of any one of the preceding Aspects, wherein the system controller is programmed to receive input indicating whether to execute the single-pair sterile connection procedure or the double-pair sterile connection procedure.

Aspect 5. The sterile connection device of any one of Aspects 1-3, wherein the system controller is programmed to determine whether to execute the single-pair sterile connection procedure or the double-pair sterile connection procedure.

Aspect 6. The sterile connection device of Aspect 5, further comprising a sensor electrically coupled to the system controller and configured to detect whether a pair of tubes is received by the second dock.

Aspect 7. The sterile connection device of any one of the preceding Aspects, wherein each cutting blade is dispensed from a single blade cartridge.

Aspect 8. The sterile connection device of any one of the preceding Aspects, wherein the first pair of tubes and the second pair of tubes are cut substantially simultaneously during the double-pair sterile connection procedure.

Aspect 9. The sterile connection device of any one of the preceding Aspects, wherein the first pair of tubes and the second pair of tubes are joined substantially simultaneously during the double-pair sterile connection procedure.

Aspect 10. A sterile connection device comprising: a housing; a first carriage including a first lower jaw defining a first portion of a first dock and a first portion of a second dock, and a first upper jaw configured to move between an open condition spaced away from the first lower jaw and a closed condition positioned adjacent to the first lower jaw; a second carriage positioned laterally of the first carriage and including a second lower jaw defining a second portion of the first dock and a second portion of the second dock, and a second upper jaw configured to move between an open condition spaced away from the second lower jaw and a closed condition positioned adjacent to the second lower jaw; a blade handling assembly configured to move cutting blades into a plurality of positions within the housing; a blade heating assembly; and a system controller programmed to execute a single-pair sterile connection procedure when a single pair of tubes is received by the first dock, no tubes are received by the second dock, a first cutting blade is associated with the first dock, a second cutting blade is associated with the second dock, and the first and second upper jaws are in the closed conditions and to execute a double-pair sterile connection procedure when a first pair of tubes is received by the first dock, a second pair of tubes is received by the second dock, the first cutting blade is associated with the first dock, the second cutting blade is associated with the second dock, and the first and second upper jaws are in the closed conditions, wherein the single-pair sterile connection procedure includes controlling the blade handling assembly, the blade heating assembly, and at least one of the first and second carriages to cut the single pair of tubes using the first cutting blade and then join the single pair of tubes, the double-pair sterile connection procedure includes controlling the blade handling assembly, the blade heating assembly, and at least one of the first and second carriages to cut the first pair of tubes using the first cutting blade and then join the first pair of tubes and to cut the second pair of tubes using the second cutting blade and then join the second pair of tubes, the system controller is further programmed to receive a single blade replacement input in connection with executing the single-pair sterile connection procedure, with the single blade replacement input instructing the system controller to control the blade handling assembly to advance a single replacement cutting blade in a longitudinal direction into association with the second dock, thereby advancing the second cutting blade in the longitudinal direction out of association with the second dock and into association with the first dock and advancing the first cutting blade in the longitudinal direction out of association with the first dock, and the system controller is further programmed to receive a double blade replacement input in connection with executing the double-pair sterile connection procedure, with the double blade replacement input instructing the system controller to control the blade handling assembly to advance a first replacement cutting blade in a longitudinal direction so as to place the first replacement cutting blade into association with the second dock, thereby advancing the second cutting blade in the longitudinal direction out of association with the second dock and into association with the first dock and advancing the first cutting blade in the longitudinal direction out of association with the first dock, and control the blade handling assembly to advance a second replacement cutting blade in the longitudinal direction so as to place the second replacement cutting blade into association with the second dock, thereby advancing the first replacement cutting blade in the longitudinal direction out of association with the second dock and into association with the first dock and advancing the second cutting blade in the longitudinal direction out of association with the first dock.

Aspect 11. The sterile connection device of Aspect 10, wherein the system controller is programmed to control the blade heating assembly to apply heat to the first cutting blade and not to the second cutting blade during the single-pair sterile connection procedure.

Aspect 12. The sterile connection device of Aspect 10, wherein the system controller is programmed to control the blade heating assembly to apply heat to the first cutting blade and to the second cutting blade during the single-pair sterile connection procedure.

Aspect 13. The sterile connection device of any one of Aspects 10-12, wherein the system controller is programmed to receive input indicating whether to execute the single-pair sterile connection procedure or the double-pair sterile connection procedure.

Aspect 14. The sterile connection device of any one of Aspects 10-12, wherein the system controller is programmed to determine whether to execute the single-pair sterile connection procedure or the double-pair sterile connection procedure.

Aspect 15. The sterile connection device of Aspect 14, further comprising a sensor electrically coupled to the system controller and configured to detect whether a pair of tubes is received by the second dock.

Aspect 16. The sterile connection device of any one of Aspects 10-15, wherein each cutting blade is dispensed from a single blade cartridge.

Aspect 17. The sterile connection device of any one of Aspects 10-16, wherein the first pair of tubes and the second pair of tubes are cut substantially simultaneously during the double-pair sterile connection procedure.

Aspect 18. The sterile connection device of any one of Aspects 10-17, wherein the first pair of tubes and the second pair of tubes are joined substantially simultaneously during the double-pair sterile connection procedure.

Aspect 19. The sterile connection device of any one of Aspects 10-18, wherein the system controller is programmed to require receipt of the single blade replacement input prior to executing the single-pair sterile connection procedure and to require receipt of the double blade replacement input prior to executing the double-pair sterile connection procedure.

Aspect 20. The sterile connection device of any one of Aspects 10-18, wherein the system controller is programmed to request the single blade replacement input or the double blade replacement input after completing the single-pair sterile connection procedure or the double-pair sterile connection procedure.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A sterile connection device (10) comprising:
a housing (12);
a first carriage (14) including
a first lower jaw (20) defining a first portion (22a) of a first dock (24) and a first portion (26a) of a second dock (28), and
a first upper jaw (18) configured to move between an open condition spaced away from the first lower jaw (20) and a closed condition positioned adjacent to the first lower jaw (20);
a second carriage (16) positioned laterally of the first carriage (14) and including
a second lower jaw (36) defining a second portion (22b) of the first dock (24) and a second portion (26b) of the second dock (28), and
a second upper jaw (34) configured to move between an open condition spaced away from the second lower jaw (36) and a closed condition positioned adjacent to the second lower jaw (36);
a blade handling assembly (40) configured to move cutting blades into a plurality of positions within the housing (12);
a blade heating assembly (54); and
a system controller (62) programmed to execute a single-pair sterile connection procedure when a single pair of tubes is received by the first dock (24), no tubes are received by the second dock (28), a first cutting blade is associated with the first dock (24), a second cutting blade is associated with the second dock (28), and the first and second upper jaws (18, 34) are in the closed conditions and to execute a double-pair sterile connection procedure when a first pair of tubes is received by the first dock (24), a second pair of tubes is received by the second dock (28), the first cutting blade is associated with the first dock (24), the second cutting blade is associated with the second dock (28), and the first and second upper jaws (18, 34) are in the closed conditions, wherein
the single-pair sterile connection procedure includes
controlling the blade handling assembly (40), the blade heating assembly (54), and at least one of the first and second carriages (14, 16) to cut the single pair of tubes using the first cutting blade and then join the single pair of tubes, and
controlling the blade handling assembly (40) to advance a single replacement cutting blade in a longitudinal direction into association with the second dock (28), thereby advancing the second cutting blade in the longitudinal direction out of association with the second dock (28) and into association with the first dock (24) and advancing the first cutting blade in the longitudinal direction out of association with the first dock (24), and
the double-pair sterile connection procedure includes
controlling the blade handling assembly (40), the blade heating assembly (54), and at least one of the first and second carriages (14, 16) to cut the first pair of tubes using the first cutting blade and then join the first pair of tubes and to cut the second pair of tubes using the second cutting blade and then join the second pair of tubes, and
controlling the blade handling assembly (40) to advance a first replacement cutting blades in a longitudinal direction so as to place the first replacement cutting blade into association with the second dock (28), thereby advancing the second cutting blade in the longitudinal direction out of association with the second dock (28) and into association with the first dock (24) and advancing the first cutting blade in the longitudinal direction out of association with the first dock (24), and
controlling the blade handling assembly (40) to advance a second replacement cutting blade in the longitudinal direction so as to place the second replacement cutting blade into association with the second dock (28), thereby advancing the first replacement cutting blade in the longitudinal direction out of association with the second dock (28) and into association with the first dock (24) and advancing the second cutting blade in the longitudinal direction out of association with the first dock (24).

2. A sterile connection device (10) comprising:
a housing (12);
a first carriage (14) including
a first lower jaw (20) defining a first portion (22a) of a first dock (24) and a first portion (26a) of a second dock (28), and
a first upper jaw (18) configured to move between an open condition spaced away from the first lower jaw (20) and a closed condition positioned adjacent to the first lower jaw (20);
a second carriage (16) positioned laterally of the first carriage (14) and including
a second lower jaw (36) defining a second portion (22b) of the first dock (24) and a second portion (26b) of the second dock (28), and
a second upper jaw (34) configured to move between an open condition spaced away from the second lower jaw (36) and a closed condition positioned adjacent to the second lower jaw (36);
a blade handling assembly (40) configured to move cutting blades into a plurality of positions within the housing (12);
a blade heating assembly (54); and
a system controller (62) programmed to execute a single-pair sterile connection procedure when a single pair of tubes is received by the first dock (24), no tubes are received by the second dock (28), a first cutting blade is associated with the first dock (24), a second cutting blade is associated with the second dock (28), and the first and second upper jaws (18, 34) are in the closed conditions and to execute a double-pair sterile connection procedure when a first pair of tubes is received by the first dock (24), a second pair of tubes is received by the second dock (28), the first cutting blade is associated with the first dock (24), the second cutting blade is associated with the second dock (28), and the first and second upper jaws (18, 34) are in the closed conditions, wherein
the single-pair sterile connection procedure includes controlling the blade handling assembly (40), the blade heating assembly (54), and at least one of the first and second carriages (14, 16) to cut the single pair of tubes using the first cutting blade and then join the single pair of tubes,
the double-pair sterile connection procedure includes controlling the blade handling assembly (40), the blade heating assembly (54), and at least one of the first and second carriages (14, 16) to cut the first pair of tubes using the first cutting blade and then join the first pair of tubes and to cut the second pair of tubes using the second cutting blade and then join the second pair of tubes,
the system controller (62) is further programmed to receive a single blade replacement input in connection with executing the single-pair sterile connection procedure, with the single blade replacement input instructing the system controller (62) to control the blade handling assembly (40) to advance a single replacement cutting blade in a longitudinal direction into association with the second dock (28), thereby advancing the second cutting blade in the longitudinal direction out of association with the second dock (28) and into association with the first dock (24) and advancing the first cutting blade in the longitudinal direction out of association with the first dock (24), and
the system controller (62) is further programmed to receive a double blade replacement input in connection with executing the double-pair sterile connection procedure, with the double blade replacement input instructing the system controller (62) to
control the blade handling assembly (40) to advance a first replacement cutting blade in a longitudinal direction so as to place the first replacement cutting blade into association with the second dock (28), thereby advancing the second cutting blade in the longitudinal direction out of association with the second dock (28) and into association with the first dock (24) and advancing the first cutting blade in the longitudinal direction out of association with the first dock (24), and
control the blade handling assembly (40) to advance a second replacement cutting blade in the longitudinal direction so as to place the second replacement cutting blade into association with the second dock (28), thereby advancing the first replacement cutting blade in the longitudinal direction out of association with the second dock (28) and into association with the first dock (24) and advancing the second cutting blade in the longitudinal direction out of association with the first dock (24).

3. The sterile connection device (10) of claim 2, wherein the system controller (62) is programmed to require receipt of the single blade replacement input prior to executing the single-pair sterile connection procedure and to require receipt of the double blade replacement input prior to executing the double-pair sterile connection procedure.

4. The sterile connection device (10) of claim 2, wherein the system controller (62) is programmed to request the single blade replacement input or the double blade replacement input after completing the single-pair sterile connection procedure or the double-pair sterile connection procedure.

5. The sterile connection device (10) of any one of the preceding claims, wherein the system controller (62) is programmed to control the blade heating assembly (54) to apply heat to the first cutting blade and not to the second cutting blade during the single-pair sterile connection procedure.

6. The sterile connection device (10) of any one of claims 1-4, wherein the system controller (62) is programmed to control the blade heating assembly (54) to apply heat to the first cutting blade and to the second cutting blade during the single-pair sterile connection procedure.

7. The sterile connection device (10) of any one of the preceding claims, wherein the system controller (62) is programmed to receive input indicating whether to execute the single-pair sterile connection procedure or the double-pair sterile connection procedure.

8. The sterile connection device (10) of any one of claims 1-6, wherein the system controller (62) is programmed to determine whether to execute the single-pair sterile connection procedure or the double-pair sterile connection procedure.

9. The sterile connection device (10) of claim 8, further comprising a sensor (67) electrically coupled to the system controller (62) and configured to detect whether a pair of tubes is received by the second dock (28).

10. The sterile connection device (10) of any one of the preceding claims, wherein each cutting blade is dispensed from a single blade cartridge.

11. The sterile connection device (10) of any one of the preceding claims, wherein the first pair of tubes and the second pair of tubes are cut substantially simultaneously during the double-pair sterile connection procedure.

12. The sterile connection device (10) of any one of the preceding claims, wherein the first pair of tubes and the second pair of tubes are joined substantially simultaneously during the double-pair sterile connection procedure.
